(19) Europäisches Patentamt
European Patent Office
Office européen des brevets

(11) Publication number: **0 041 587 B1**

(12) **EUROPEAN PATENT SPECIFICATION**

(45) Date of publication of patent specification: **14.11.84**

(21) Application number: **80301897.7**

(22) Date of filing: **06.06.80**

(51) Int. Cl.³: **B 01 J 31/20**, B 01 J 31/16, C 07 C 45/49, C 07 C 47/02, C 07 C 120/00

(54) **Catalyst compositions, production thereof, and use in production of oxygenated organic compounds.**

(43) Date of publication of application:
**16.12.81 Bulletin 81/50**

(45) Publication of the grant of the patent:
**14.11.84 Bulletin 84/46**

(84) Designated Contracting States:
**BE CH DE FR GB IT LI LU NL SE**

(56) References cited:
**DE-A-2 901 347**
**FR-A-2 394 552**
**GB-A-2 028 677**
**US-A-4 189 448**

(73) Proprietor: **THE STANDARD OIL COMPANY**
**Midland Building**
**Cleveland, Ohio 44115 (US)**

(72) Inventor: **Pesa, Frederick Anthony**
**764 Circlewood Drive**
**Aurora, Ohio 44202 (US)**
Inventor: **Haase, Thomas Alan**
**2328 Allison Road**
**University Heights, Ohio 44118 (US)**

(74) Representative: **Smith, Sydney (GB) et al**
**Elkington & Fife 52/54 High Holborn**
**GB-London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new catalyst compositions, to the production thereof and their use in the production of oxygenated organic compounds.

There are several known methods for producing oxygenated organic compounds. The acid catalyzed ($H_2SO_4$, $HBF_4$ etc.) synthesis of carboxylic acids or esters by the reaction of an olefinic substrate with CO and water or alcohol has been known since 1931. (J. Falbe, "Carbon Monoxide in Organic Synthesis", Springer-Verlag, New York (1970)). Although this process was used on a commercial scale it does have serious limitations due to the reaction conditions and the isomeric composition of the products.

A more commercially important synthesis of carboxylic acid/esters is the direct carbonylation of olefinic substrates with CO and water/alcohol conducted in the presence of transition metals. In general, this carbonylation reaction, discovered by Repp in 1940 (I. Wender and P. Pino, "Organic Synthesis Via Metal Carbonyls", Volume 2, John Wiley, New York (1977)), involves the addition of carbon monoxide, carboxyl alkyl or amide group (Y—H where Y equals —OR or —NHR and R is an alkyl), and an olefin.

However, when an unsymmetrical olefin is used as the substrate at least two isomeric products are obtained. No general method has been developed for the control of the isomeric product composition.

The present invention overcomes some of these problems present in the prior art. For example, the invention process results in higher conversions, higher yields and faster reaction rates than those disclosed in the prior art. Furthermore, the instant process allows one to obtain a high yield of a particular isomeric product composition. Thus, extremely high selectivities of particular oxygenated organic compounds can be obtained by the process of the invention.

Finally, the prior art carbonylation reactions operate under extreme conditions of temperature and pressure. In general, temperatures in the range of 160°C to 300°C and pressures in the range of 1,500 to 5,000 psi (105—350 kg/cm²) are required. On the other hand, the process according to the invention may be carried out under relatively mild conditions of temperature and pressure. This further advantage can result in substantial cost savings in the production of oxygenated organic compounds.

In DE—A—2901347 there is described a process of hydroformylation involving reacting an ethylenically unsaturated compound with carbon monoxide and hydrogen in the presence of a catalyst wherein the catalyst is one which has been prepared by reaction between a cobalt carbonyl compound and a nitrogen-containing heterocyclic compound with an enolic hydroxyl group on that carbon atom adjacent to the ring-forming nitrogen atom.

In GB—A—2,028,677 there is described a process of carbonylating olefinic compounds in which the olefinic compound is reacted with carbon monoxide and hydrogen or an alcohol in the presence of a cobalt carbonyl catalyst together with a secondary phosphine oxide having the general formula

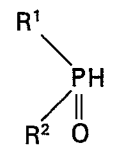

It has now been discovered that oxygenated organic compounds can be produced by contacting an olefinically unsaturated compound with carbon monoxide and a compound containing a replaceable hydrogen atom in the presence of a new catalyst composition comprising cobalt and/or ruthenium carbonyl and a promoter ligand which belongs to a defined class of heterocyclic nitrogen compounds or to a defined class of phosphorus or sulphur oxides.

In particular, the process of the invention results in high yields of oxygenated organic compounds when operating at much lower temperatures and pressures than disclosed in the prior art. In addition, the product distribution can be varied significantly by changing the $CO/H_2$ ratio, pressure, ligand, solvent, reaction time and other process variables.

Thus, the present invention provides a new catalyst which comprises a specified promoter ligand and at least one of cobalt and ruthenium carbonyl. In addition the invention provides a novel process for the production of an oxygenated organic compound comprising contacting an olefinically unsaturated compound with carbon monoxide and a compound containing a replaceable hydrogen atom in the presence of the new catalyst. The present invention also provides a novel process for the production of an oxygenated organic compound comprising contacting an olefinically unsaturated compound containing an alcohol moiety with carbon monoxide in the presence of the new catalyst.

In a specific example of the invention the carbonylation reaction of acrylonitrile, carbon monoxide, hydrogen gas, and methanol to yield methyl-$\beta$-cyanopropionate proceeds smoothly using a catalyst comprising cobalt carbonyl and a heterocyclic nitrogen oxide promoter ligand of a specified class.

According to the present invention, improved yields and selectivities of oxygenated organic compounds are obtained by contacting an olefinically unsaturated compound with carbon monoxide

and a compound containing a replaceable hydrogen atom over a catalyst comprising cobalt and/or ruthenium carbonyl and a promoter ligand selected from heterocyclic nitrogen compounds as defined below and phosphorus or sulphur oxides as defined below. The overall reaction taking place in this process is represented by the following equation:

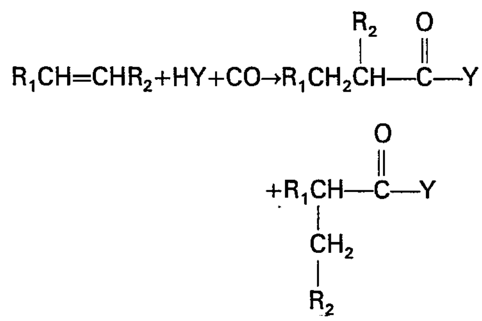

$R_1$, $R_2$ and Y are defined below.

Reactants

Olefinically unsaturated compounds which can be employed as reactants in the process of the invention have the following structure:

$$R_{11}CH{=}CHR_{12}$$

wherein $R_{11}$ and $R_{12}$ are each independently selected from:

(1) hydrogen (either $R_{11}$ or $R_{12}$ but not both);
(2) $C_{1-30}$ alkyl;
(3) —$(CH_2)_p$—CN, wherein p is 0—3; and
(4) —$(CH_2)_q$—$OR_{13}$, wherein q is 1—30 and $R_{13}$ is hydrogen or methyl.

Preferably, the olefinically unsaturated compounds comprise compounds wherein $R_{11}$ and $R_{12}$ are each independently:

(1) hydrogen (either $R_{11}$ or $R_{12}$ but not both);
(2) $C_{1-10}$ alkyl;
(3) —$(CH_2)_p$—CN, wherein p is 0—2; and
(4) —$(CH_2)_q$—OH, wherein q is 1—10.

Most preferably, the olefinically unsaturated compounds comprise compounds wherein $R_1$ and $R_2$ are each independently selected from hydrogen (either $R_1$ or $R_2$ but not both), methyl and —$(CH_2)_p$—CN, wherein p is 0—1.

The second component in the inventive reaction system is a compound containing a replaceable hydrogen atom. This compound can be represented by the following formula:

$$H{-}Y$$

wherein Y is selected from:

(1) $OR_{14}$ wherein $R_{14}$ is a $C_{1-30}$ alkyl;

(2) 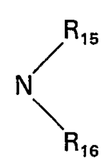

wherein $R_{15}$ and $R_{16}$ are each independently selected from $C_{1-10}$ alkyls; and
(3) H.

Preferably Y is selected from:

(1) $OR_{14}$ wherein $R_{14}$ is a $C_{1-10}$ alkyl;

(2) 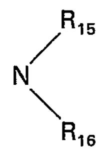

wherein $R_{15}$ and $R_{16}$ are each independently selected from $C_{1-4}$ alkyls; and
(3) H.

More preferably Y is selected from:

(1) $OR_{14}$ wherein $R_{14}$ is a $C_{1-4}$ alkyl; and
(2) H.

The second component is most preferably either methanol or hydrogen.

In the embodiment of the invention in which H—Y is an alcohol or amine, it is preferred to add hydrogen gas to the reaction system. Preferably the amount of hydrogen gas so added comprises less than 10% by volume of the total amount of the hydrogen gas and carbon monoxide gas in the reaction system. More preferably the hydrogen gas comprises 0.5% to 7.5% by volume of the hydrogen and carbon monoxide gas. The addition of hydrogen gas can increase both the yield and selectivity to desired products in this mode of the invention.

When $H_2$ is the compound containing a replaceable hydrogen atom then the reaction system will preferably contain 10% to 60% by volume hydrogen gas based on the total volume of the carbon monoxide and hydrogen gas. More preferably the reaction system will contain about 50% hydrogen gas.

One way to supply carbon monoxide and hydrogen gas into the reaction system is in the form of synthesis gas. The amount of hydrogen in the synthesis gas can be easily adjusted prior to insertion into the reactor.

The amount of carbon monoxide in the reaction system is not critical. Preferably the carbon monoxide is present in at least stoichiometric amounts and most preferably the carbon monoxide is present in amounts greatly in excess of stoichiometric amounts. If desired, a carrier gas which is inert to the reactants, products and catalyst can be included in the reaction system.

The molar ratio of the compound containing a replaceable hydrogen atom to the olefinically unsaturated compound can be 0.5—100:1 with a ratio of 1—10:1 being preferred. This ratio does not include the hydrogen gas which may be added to the reaction system when H—Y is an alcohol or amine.

In the embodiment of the invention in which the olefin reactant is an alcohol (i.e. wherein $R_1$ or $R_2$ is $-(CH_2)_q-OH$), it has been found that the terminal hydrogen atom on the alcohol group will itself serve as a replaceable hydrogen atom. As a result, the alcohol moiety of the olefin will react with the olefinic double bond of the olefin thereby producing a lactone. In this reaction system no H—Y component need be included since the olefin itself acts both as the olefin and the H—Y component. The reaction in this particular system is shown by the following equation:

$$R_1CH{=}CHR_2 + CO \xrightarrow{\text{Cat.}} \text{Lactone}$$

wherein at least one of $R_1$ and $R_2$ is an alcohol.

Process conditions

Generally, in carrying out the process of the invention, the olefinically unsaturated compound, carbon monoxide, and the compound containing a replaceable hydrogen atom are contacted with one another in the liquid phase in the presence of the catalyst described below. The reaction can be accomplished in the batch mode or continuously.

The reaction temperature is normally maintained between 50°C to 150°C and most preferably at about 100°C. The reaction pressure is normally maintained at 7 to 175 kg/cm$^2$ (100 to 2500 psi) preferably at 49 to 70 kg/cm$^2$ (700 to 1000 psi). When the reaction is carried out in a batch mode, the reactants and catalyst are contacted with one another for a period of ten minutes through six hours, and preferably one half hour to four hours. A reaction time of less than ten minutes or more than six hours can be used if desired although better results will be obtained if the reaction time is maintained within this range. When the process is carried out on a continuous basis, the reaction catalyst contact time is normally 10 seconds to 10 minutes, preferably 100 seconds to 5 minutes.

Both the rate of reaction and product distribution can be varied significantly by changing the process parameters. For example, normally an increase in pressure increases the rate of reaction. However, at very high pressures the reaction rate may decrease due to catalyst decomposition. Furthermore, the selectivity to a particular product may be affected by pressure changes, e.g. in the

carbonylation of acrylonitrile there is an increase in the selectivity to the n-cyanoester (3CE) as the pressure decreases.

A balance exists between temperatures and pressure with respect to catalyst decomposition. Generally, as the temperature increases the rate of reaction increases. However, an anomalous effect may occur due to partial catalyst decomposition. Thus, the temperature and pressure must be carefully adjusted.

Similarly, residence time has a large effect on homogeneous processes. For example, in the process for the carbonylation of acrylonitrile, the selectivity to methyl-$\beta$-cyanopropionate is much higher at short reaction times. Applicants surmise that the reduction in selectivity as a function of reaction time is caused by the reduction of the ligand, e.g. 4-picoline-N-oxide is reduced to 4-picoline. In view of the above discussion, it is clear that a particular reaction rate and product distribution can be obtained by a careful adjustment of the process variables.

Catalysts

The catalyst employed in the inventive process can be generally described as one of two types. Both types comprise cobalt and/or ruthenium carbonyl and a promoter ligand in an organic solvent. The promoter ligand is either a heterocyclic nitrogen compound or a phosphorus or sulphur oxide.

The nitrogen heterocyclic promoter ligand has the following structure:

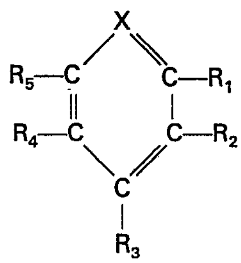

wherein X is NO; and wherein $R_1$, $R_2$, $R_3$, $R_4$, and $R_5$ are each independently selected from:

(1) H;
(2) $C_{1-10}$ alkyls;
(3) $(CH_2)_qOH$ wherein q is 0—10;

(4) 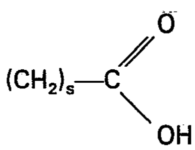

wherein s is 0—10; and
(5) $O(CH_2)_tCH_3$ wherein t is 0—10;

wherein $R_1$ and $R_2$ may comprise a five to eight membered carbocyclic fused ring optionally substituted with $C_{1-10}$ alkyls.

Preferably, $R_1$, $R_2$, $R_3$, $R_4$, and $R_5$ are selected from H, $C_{1-4}$ alkyls, $(CH_2)_qOH$, wherein q is 0 or 1

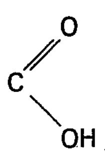,

and $OCH_3$. Most preferably, $R_1$, $R_2$, $R_3$, $R_4$, and $R_5$ are selected from H, $CH_3$, and $OCH_3$.

The phosphorus or sulphur oxide promoter ligands having the following formulae:

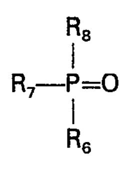 and 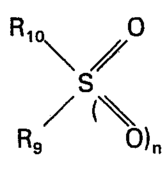

wherein $R_6$, $R_7$, $R_8$, $R_9$, and $R_{10}$ are each independently selected from:

(1) $C_{1-10}$ alkyls;
(2) polynuclear aryls containing up to 12 carbon atoms, optionally substituted with $C_{1-10}$ alkyls; and
(3) $O(CH_2)_tCH_3$, wherein t is 0—10; and wherein n is 0 or 1.

Preferably, $R_6$, $R_7$, $R_8$, $R_9$, and $R_{10}$ are each independently selected from $C_{1-4}$ alkyls; aryls, optionally substituted with $C_1$—$C_4$ alkyls and $OCH_3$. Most preferably $R_6$, $R_7$, $R_8$, $R_9$, and $R_{10}$ are $CH_3$.

The catalyst according to the invention can be prepared by mixing the cobalt and/or ruthenium carbonyl with at least one promoter ligand in a solvent. The cobalt and/or ruthenium carbonyl and the promoter ligand may be added simultaneously or separately to the solvent. The exact relationship in the solvent between the cobalt and/or ruthenium carbonyl and the promoter ligand is not known.

Any solvent in which the catalyst is soluble may be used in the present invention. Preferably, the solvent is an alcohol, aromatic, ester, nitrile and/or dinitrile. The solvent is most preferably an alcohol or ester. In fact, the alcohol can be both a compound containing a replaceable hydrogen atom described above and the solvent. The preferred catalyst concentration in the solvent is normally between 0.1% to 5% by weight.

The cobalt and/or ruthenium carbonyl can be added to the solvent in any form from which cobalt and/or ruthenium carbonyl could be formed. For example, it is well known in the art that carbonyls can be formed from naphthalates, salts and nitrates and thus suitable naphthalates, salts and nitrates can be added to the solvent to form a carbonyl compound *in situ*. Preferably, the catalyst contains cobalt carbonyl.

In general, the promoter ligand to cobalt and/or ruthenium carbonyl molar ratio is 0.1—50:1, preferably about 0.5—4, and more preferably about 2:1. This ratio will vary depending on the promoter ligand chosen. At high ligand to carbonyl ratios (i.e. 4:1) the rate of reaction substantially decreases even though selectivity to the desired product may be increased. Thus, in the carbonylation of acrylonitrile, at high ligand/carbonyl ratios the reaction rate decreases but the selectivity to methyl-$\beta$-cyanopropionate increases.

The catalyst of this invention is dissolved in the reaction medium as a homogeneous catalyst. These homogeneous catalysts are prepared by known techniques. Specific preparations of these catalysts are shown in the working examples of this specification. Broadly, however, the catalysts of this invention can be prepared by any of the techniques known in the art.

Recovery

The reaction product obtained upon completion of the reaction is normally in the form of a liquid and composed primarily of unreacted reactants, catalyst and oxygenated organic compounds. This reaction product can be subjected to suitable known separation techniques, i.e. solvent extraction and fractional distillation, to yield the desired end products.

A particularly good method for separating the catalyst from the products obtained in the present process is by the use of conjugate phase extraction. In this separation scheme, the reaction effluent is treated with a $C_5$ to $C_8$ hydrocarbon which is miscible with the reaction solvent but which is a very poor solvent for the catalyst. Examples of such hydrocarbons are pentane, hexane and octane. Enough of this hydrocarbon is added to the reactor effluent to separate almost all of the catalyst into one phase and a significant amount of products into the other phase. Generally, this is between 1 to 4 volumes of hydrocarbon per volume of reactor effluent.

It is desirable to exclude oxygen from this separation system so that catalyst decomposition will not occur. It is also desirable to minimize the amounts of unreacted substrates in the reactor effluent prior to treatment with the hydrocarbon. This can be accomplished by simple distillation or vacuum stripping. Finally, it is desirable to separate the products and reactants as quickly as possible to reduce the possibility of unwanted side reactions, e.g. methyl-$\beta$-cyanopropionate reacts with acrylonitrile to produce a dicyano ester.

The catalyst containing hydrocarbon phase can be diluted and recycled back to the reactor. The product phase is then subjected to known separation techniques such as distillation or extraction.

The oxygenated organic compounds produced by this process are useful as precursors to polymers. The esters are also useful in perfumes, flavourings and pharmaceuticals. The aldehydes are useful as plasticizers and as intermediates for alcohols.

In order more thoroughly to illustrate the present invention, the following examples are given. In these examples, the following definitions are used:

$$\%\ \text{Conv} = \frac{\text{moles carbon in reactant converted to product}}{\text{moles carbon in reactant fed}} \times 100$$

$$\%\ \text{Yield} = \frac{\text{moles carbon of olefinically unsaturated compound converted to product}}{\text{moles carbon of olefinically unsaturated compound fed}} \times 100$$

The results have all been adjusted to a 100% carbon balance.

In general, the experimental method consists of placing a pre-weighed solution of olefinically unsaturated compound, promoter ligand, compound containing a replaceable hydrogen atom and solvent into a glasslined autoclave. Next, cobalt carbonyl, $Co_2(CO)_8$, is added and the autoclave sealed.

The autoclave is flushed twice with synthesis gas and then charged with the synthesis gas to the desired pressure. The temperature is then increased and the reaction proceeds for 1 to 4 hours. Occasionally, samples are withdrawn during the course of the reaction through the vent tube and subjected to gas chromatography analysis. After the runs, the glasslined autoclave is brought to room temperature by cooling with cold water, depressurized and opened for product analysis.

The results of the experiments are shown in Table I. A glossary of terms follows Table I and specifies the meanings of the abbreviations used in Table I.

Example 1

13.5 g of acrylonitrile, 0.88 g of 4-picoline-N-oxide, 9.78 g of methanol and 100 ml of adiponitrile were placed in a glasslined autoclave. Next, 1.37 g of $Co_2(CO)_8$ were added and the autoclave sealed.

The autoclave was charged with synthesis gas containing 5% $H_2$ until a pressure of 70 kg/cm$^2$ (1,000 psi) was reached. The temperature was set at 97.5°C and the reaction proceeded for 90 minutes. The autoclave was then brought to room temperature by cooling with cold water, depressurized and opened for product analysis. The product analysis is shown in Table I.

Example 2

13.5 g of acrylonitrile, 0.88 g of 4-picoline-N-oxide and 100 ml of methanol were placed in a glasslined autoclave. Next, 1.37 g of $Co_2(CO)_8$ were added and the autoclave sealed.

The autoclave was charged with synthesis gas containing 5% $H_2$ to a pressure of 70 kg/cm$^2$ (1,000 psi). The temperature was set at 97.5°C and the reaction proceeded for 150 minutes. The autoclave was then brought to room temperature by cooling with cold water, depressurized and opened for product analysis. The product analysis is shown in Table I.

Examples 3—80

The procedure outlined in Example 1 was followed with the molar ratio of cobalt carbonyl/ligand, temperature, pressure, solvent and reaction time being varied. These variables are specified in Table I for each example. Table I also shows the product analysis for Examples 3—80.

Example 81

13.5 g of acrylonitrile, 0.75 g of pyridine-N-oxide and 100 ml of methanol were placed into a glasslined autoclave. Next, 1.37 g of $Co_2(CO)_8$ were added and the autoclave sealed.

The autoclave was charged with synthesis gas containing 5% $H_2$ to a pressure of 56 kg/cm$^2$ (800 psi). The temperature was set at 95°C and the reaction proceeded for 60 minutes. The glasslined autoclave was then brought to room temperature by cooling with cold water, depressurized and opened for product analysis. The product analysis is shown in Table I.

Example 82

This example followed the same procedure outlined in Example 81 except that the temperature and reaction time were varied as set given in Table I. The results are shown in Table I.

Example 83

13.5 g of acrylonitrile, 0.88 g of 2-picoline-N-oxide and 100 ml of methanol were placed in a glasslined autoclave. Next, 1.37 g of $Co_2(CO)_8$ were added and the autoclave sealed.

The autoclave was charged with synthesis gas containing 5% $H_2$ to a pressure of 56 kg/cm$^2$ (800 psi). The temperature was set at 95°C and the reaction proceeded for 120 minutes. The glasslined autoclave was then brought to room temperature by cooling with cold water, depressurized and opened for product analysis. The product analysis is shown in Table I.

Examples 84—97

The procedure outlined in Example 83 was followed except that the molar ratio of cobalt carbonyl/ligand, solvent, ligand, temperature, pressure and reaction time were varied. These variables are specified in Table I for each example. Table I also shows the product analysis for Examples 84—97.

TABLE I

Catalyst: $Co_2(CO)_8$+Ligand
Unsaturated Olefin Feed: Acrylonitrile

| Example | Ligand | $Co_2(CO)_8$/ Ligand | Solvent | Temp (°C) | Pres (kg/cm$^2$) | Time (min) | Conv (%) |
|---|---|---|---|---|---|---|---|
| 1 | 4-PcNox | 1:2 | ADN+MeOH | 97.5 | 70 | 90 | 84.40 |
| 2 | 4-PcNox | 1:2 | MeOH | 97.5 | 70 | 150 | 100.00 |
| 3 | 4-PcNox | 1:2 | ADN+MeOH | 97.5 | 70 | 180 | 80.80 |
| 4 | 4-PcNox | 1:2 | MeOH | 75 | 56 | 180 | 100.00 |
| 5 | 4-PcNox | 1:2 | DMP+MeOH | 77.5 | 56 | 10 | 66.30 |
| 6 | 4-PcNox | 1:2 | DMP+MeOH | 77.5 | 56 | 60 | 100.00 |
| 7 | 4-PcNox | 1:2 | MeOH | 95 | 56 | 60 | 100.00 |
| 8 | 4-PcNox | 1:4 | MeOH | 95 | 56 | 60 | 100.00 |
| 9 | 4-PcNox | 1:1 | MeOH | 95 | 56 | 90 | 97.10 |
| 10 | 4-PcNox | 1:2 | DMP+MeOH | 97.5 | 56 | 15 | 98.60 |
| 11[1] | 4-PcNox | 1:2 | DMP+MeOH | 97.5 | 56 | 15 | 51.50 |
| 12[2] | 4-PcNox | 1:2 | DMP+MeOH | 97.5 | 56 | 20 | 99.50 |
| 13 | 4-PcNox | 1:2 | DMP+MeOH | 97.5 | 56 | 20 | 51.73 |
| 14 | 4-PcNox | 1:1 | DMP+MeOH | 97.5 | 56 | 25 | 99.90 |
| 15 | 4-PcNox | 1:2 | DMP+MeOH | 97.5 | 56 | 30 | 70.49 |
| 16 | 4-PcNox | 1:4 | DMP+MeOH | 97.5 | 56 | 35 | 99.10 |
| 17[1] | 4-PcNox | 1:2 | DMP+MeOH | 97.5 | 56 | 40 | 100.00 |
| 18[1] | 4-PcNox | 1:2 | DMP+MeOH | 97.5 | 56 | 45 | 99.95 |
| 19 | 4-PcNox | 1:2 | DMP+MeOH | 97.5 | 56 | 60 | 98.90 |
| 20[3] | 4-PcNox | 1:2 | MeOH | 97.5 | 56 | 60 | 100.00 |
| 21 | 4-PcNox | 1:2 | Benzene+ MeOH | 97.5 | 56 | 60 | 79.88 |
| 22 | 4-PcNox | 2:1 | MeOH | 97.5 | 56 | 90 | 100.00 |
| 23 | 4-PcNox | 1:4 | MeOH | 97.5 | 56 | 90 | 100.00 |
| 24 | 4-PcNox | 1:8 | DMP+MeOH | 97.5 | 56 | 115 | 99.90 |

TABLE I (contd.)

| | Yields (%) | | | | | | |
|---|---|---|---|---|---|---|---|
| Example | 3-CE | 2-CE | PN | 3-CPA | 3-CPAA | 3-MPN | Other |
| 1 | 97.50 | — | — | — | — | 2.50 | — |
| 2 | 54.10 | 38.90 | 1.69 | — | 4.47 | — | 0.82 |
| 3 | 62.90 | 35.50 | — | — | — | 1.60 | — |
| 4 | 40.10 | 57.60 | — | — | 0.78 | — | 0.62 |
| 5 | 57.00 | 34.50 | 1.40 | — | 0.20 | 6.70 | — |
| 6 | 52.40 | 41.10 | 0.90 | — | 1.50 | 4.20 | — |
| 7 | 55.30 | 40.24 | 1.33 | — | 2.25 | — | 0.90 |
| 8 | 51.26 | 45.98 | 1.16 | — | 0.64 | 0.61 | 0.35 |
| 9 | 51.80 | 42.52 | 1.53 | — | 3.39 | — | 0.75 |
| 10 | 53.40 | 40.80 | 0.62 | — | 0.23 | 4.90 | 0.00 |
| 11[1] | 50.90 | 39.20 | 3.20 | — | — | 6.80 | — |
| 12[2] | 47.40 | 33.80 | 1.90 | 11.23 | 1.73 | 3.90 | — |
| 13 | 90.47 | — | — | — | — | 9.53 | — |
| 14 | 40.50 | 40.50 | — | 8.80 | 6.80 | 0.32 | — |
| 15 | 91.50 | — | — | — | — | 7.94 | 0.56 |
| 16 | 52.30 | 38.70 | 0.50 | — | 0.40 | 8.10 | — |
| 17[1] | 50.60 | 46.20 | — | — | — | 3.10 | — |
| 18[1] | 51.30 | 44.30 | 0.50 | — | 0.50 | 3.40 | — |
| 19 | 45.00 | 52.00 | 2.60 | — | — | 2.40 | 0.02 |
| 20[3] | 54.50 | 38.70 | 2.00 | — | 2.00 | — | 2.70 |
| 21 | 34.23 | 53.70 | 6.74 | — | 0.52 | 4.89 | — |
| 22 | 40.20 | 45.20 | 1.98 | — | 12.00 | 0.30 | 0.26 |
| 23 | 51.75 | 43.54 | 1.46 | — | 0.80 | — | 2.45 |
| 24 | 67.70 | 21.60 | — | — | 0.62 | 10.10 | — |

TABLE I (contd.)

Catalyst: $Co_2(CO)_8$+Ligand
Unsaturated Olefin Feed: Acryonitrile

| Example | Ligand | $Co_2(CO)_8$/ Ligand | Solvent | Temp (°C) | Pres (kg/cm²) | Time (min) | Conv (%) |
|---|---|---|---|---|---|---|---|
| 25 | 4-PcNox | 2:1 | MeOH | 97.5 | 56 | 120 | 100.00 |
| 26 | 4-PcNox | 1:2 | MeOH | 97.5 | 56 | 120 | 86.80 |
| 27 | 4-PcNox | 1:16 | DMP+MeOH | 97.5 | 56 | 120 | 16.20 |
| 28 | 4-PcNox | 1:8 | ADN+MeOH | 97.5 | 56 | 150 | 22.60 |
| 29 | 4-PcNox | 1:1 | ADN+MeOH | 97.5 | 56 | 180 | 84.40 |
| 30[1] | 4-PcNox | 1:2 | MeOH | 97.5 | 56 | 180 | 100.00 |
| 31[4] | 4-PcNox | 1:2 | MeOH | 97.5 | 56 | 180 | 48.10 |
| 32[5] | 4-PcNox | 1:2 | MeOH | 97.5 | 56 | 180 | 93.40 |
| 33[6] | 4-PcNox | 1:2 | MeOH | 97.5 | 56 | 180 | 100.00 |
| 34[4] | 4-PcNox | 1:2 | DMP+MeOH | 97.5 | 56 | 180 | 91.10 |
| 35 | 4-PcNox | 1:8 | MeOH | 97.5 | 56 | 180 | 100.00 |
| 36 | 4-PcNox | 1:16 | MeOH | 97.5 | 56 | 180 | 100.00 |
| 37 | 4-PcNox | 1:2 | MeOH | 97.5 | 56 | 225 | 100.00 |
| 38[7] | 4-PcNox | 1:2 | MeOH | 97.5 | 56 | 300 | 53.70 |
| 39 | 4-PcNox | 1:4 | MeOH | 115 | 56 | 120 | 100.00 |
| 40 | 4-PcNox | 1:2 | DMP+MeOH | 117.5 | 56 | 10 | 87.70 |
| 41 | 4-PcNox | 1:2 | DMP+MeOH | 117.5 | 56 | 60 | 100.00 |
| 42 | 4-PcNox | 1:2 | MeOH | 125 | 56 | 180 | 100.00 |
| 43 | 4-PcNox | 1:2 | MeOH | 97.5 | 49 | 50 | 100.00 |
| 44 | 4-PcNox | 1:2 | ADN+MeOH | 97.5 | 42 | 150 | 57.60 |
| 45 | 4-PcNox | 1;2 | MeOH | 97.5 | 42 | 180 | 100.00 |
| 46 | 4-PcNox | 1:2 | DMP+MeOH | 97.5 | 35 | 120 | 99.70 |
| 47[8] | 4-PcNox | 1:2 | DMP+MeOH | 85 | 28 | 170 | 74.60 |
| 48[8] | 4-PcNox | 1:2 | DMP+MeOH | 85 | 28 | 420 | 97.30 |

TABLE I (contd.)

| | Yields (%) | | | | | | |
|---|---|---|---|---|---|---|---|
| Example | 3-CE | 2-CE | PN | 3-CPA | 3-CPAA | 3-MPN | Other |
| 25 | 40.40 | 47.50 | 2.40 | — | 8.72 | 0.57 | 0.41 |
| 26 | 57.80 | 29.50 | 5.20 | — | 3.60 | 1.30 | 2.30 |
| 27 | 64.80 | — | — | — | 0.25 | 34.90 | — |
| 28 | 53.70 | 20.70 | — | — | — | 25.60 | — |
| 29 | 68.90 | 23.50 | 2.00 | — | 2.30 | 3.30 | — |
| 30[1] | 56.30 | 39.60 | 1.29 | — | 1.13 | 0.20 | 1.49 |
| 31[4] | 9.73 | — | — | — | — | 84.20 | 6.04 |
| 32[5] | 22.90 | 74.00 | 1.50 | — | — | — | 1.50 |
| 33[6] | 48.90 | 48.00 | 1.20 | — | 0.30 | 0.80 | 0.80 |
| 34[4] | 39.90 | 53.90 | Trace | — | 0.40 | 5.30 | — |
| 35 | 36.90 | 60.60 | 1.01 | — | 0.25 | 0.50 | 0.70 |
| 36 | 26.80 | 48.30 | 10.46 | — | — | 14.33 | 0.40 |
| 37 | 30.90 | 61.90 | 1.00 | — | 3.50 | 2.70 | — |
| 38[7] | 57.00 | 39.00 | — | — | 2.40 | 1.00 | 0.50 |
| 39 | 56.93 | 37.52 | 1.43 | — | 3.13 | — | 0.99 |
| 40 | 67.00 | 18.70 | 4.20 | — | 0.20 | 9.90 | — |
| 41 | 52.40 | 38.20 | 1.90 | — | 2.80 | 4.60 | — |
| 42 | 49.30 | 40.00 | 4.48 | — | 4.49 | — | 1.76 |
| 43 | 57.00 | 35.50 | 0.80 | — | 5.20 | 1.5 | Trace |
| 44 | 99.80 | — | — | — | — | 0.20 | — |
| 45 | 56.30 | 37.80 | 2.01 | — | 2.21 | 1.15 | 0.54 |
| 46 | 55.90 | 40.30 | 1.50 | — | 0.60 | — | 1.60 |
| 47[8] | 60.80 | 35.10 | — | — | — | 3.90 | 0.19 |
| 48[8] | 51.50 | 38.40 | 0.90 | — | 3.60 | 3.41 | 2.24 |

TABLE I (contd.)

Catalyst: $Co_2(CO)_9$+Ligand
Unsaturated Olefin Feed: Acrylonitrile

| Example | Ligand | $Co_2(CO)_8$/ Ligand | Solvent | Temp (°C) | Pres (kg/cm$^2$) | Time (min) | Conv (%) |
|---|---|---|---|---|---|---|---|
| 49 | 4-PcNox | 1:2 | DMP+MeOH | 97.5 | 28 | 25 | 88.20 |
| 50 | 4-PcNox | 1:4 | DMP+MeOH | 97.5 | 28 | 25 | 51.51 |
| 51 | 4-PcNox | 1:2 | DMP+MeOH | 97.5 | 28 | 40 | 97.30 |
| 52 | 4-PcNox | 1:4 | DMP+MeOH | 97.5 | 28 | 50 | 76.03 |
| 53 | 4-PcNox | 1:2 | DMP+MeOH | 97.5 | 28 | 60 | 100.00 |
| 54 | 4-PcNox | 1:4 | DMP+MeOH | 97.5 | 28 | 60 | 67.95 |
| 55 | 4-PcNox | 1:4 | DMP+MeOH | 97.5 | 28 | 150 | 91.95 |
| 56[8] | 4-PcNox | 1:2 | DMP+MeOH | 97.5 | 28 | 180 | 49.70 |
| 57 | 4-PcNox | 1:2 | DMP+MeOH | 97.5 | 28 | 285 | 55.70 |
| 58 | 4-PcNox | 1:2 | DMP+MeOH | 97.5 | 28 | 300 | 62.12 |
| 59 | 4-PcNox | 1:8 | DMP+MeOH | 117.5 | 28 | 120 | 47.50 |
| 60 | 4-PcNox | 1:2 | DMP+MeOH | 97.5 | 21 | 15 | 69.70 |
| 61 | 4-PcNox | 1:2 | DMP+MeOH | 97.5 | 21 | 20 | 24.40 |
| 62 | 4-PcNox | 1:1 | DMP+MeOH | 97.5 | 21 | 25 | 85.60 |
| 63 | 4-PcNox | 1:2 | DMP+MeOH | 97.5 | 21 | 25 | 46.80 |
| 64 | 4-PcNox | 1:2 | DMP+MeOH | 97.5 | 21 | 30 | 80.40 |
| 65 | 4-PcNox | 1:2 | DMP+MeOH | 97.5 | 21 | 50 | 59.80 |
| 66 | 4-PcNox | 1:2 | DMP+MeOH | 97.5 | 21 | 60 | 66.30 |
| 67 | 4-PcNox | 1:2 | DMP+MeOH | 97.5 | 21 | 70 | 72.70 |
| 68 | 4-PcNox | 1:2 | DMP+MeOH | 97.5 | 21 | 90 | 79.10 |
| 69 | 4-PcNox | 1:2 | DMP+MeOH | 97.5 | 21 | 120 | 81.20 |
| 70 | 4-PcNox | 1:2 | DMP+MeOH | 97.5 | 21 | 140 | 88.70 |
| 71 | 4-PcNox | 1:2 | DMP+MeOH | 97.5 | 21 | 150 | 91.90 |
| 72 | 4-PcNox | 1:8 | DMP+MeOH | 97.5 | 21 | 150 | 85.70 |

TABLE I (contd.)

| | Yields (%) | | | | | | |
|---|---|---|---|---|---|---|---|
| Example | 3-CE | 2-CE | PN | 3-CPA | 3-CPAA | 3-MPN | Other |
| 49 | 73.70 | 17.70 | 2.80 | — | — | 5.80 | — |
| 50 | 82.36 | 7.95 | — | — | — | 9.13 | 0.56 |
| 51 | 70.90 | 21.30 | 2.10 | — | 0.40 | 5.30 | — |
| 52 | 88.95 | 3.66 | — | — | — | 6.99 | 0.41 |
| 53 | 62.90 | 29.70 | 2.10 | — | 0.80 | 4.40 | — |
| 54 | 81.56 | 11.36 | — | — | — | 6.68 | 0.40 |
| 55 | 81.01 | 11.16 | 0.81 | — | — | 6.67 | 0.35 |
| 56[8] | 92.30 | 0.10 | — | — | — | 5.32 | 2.30 |
| 57 | 89.27 | 0.63 | 5.35 | — | — | 4.53 | 0.22 |
| 58 | 86.80 | 0.73 | 5.42 | — | 1.53 | 4.61 | 0.87 |
| 59 | 74.56 | 0.17 | — | — | 0.23 | 19.95 | 5.09 |
| 60 | 89.90 | — | 4.30 | — | 0.70 | 5.00 | — |
| 61 | 86.90 | — | — | — | — | 5.00 | 8.10 |
| 62 | 87.60 | — | 6.80 | — | — | 5.70 | — |
| 63 | 93.80 | — | — | — | — | 6.20 | — |
| 64 | 90.10 | — | 3.60 | — | — | 6.31 | — |
| 65 | 91.90 | — | 2.60 | — | — | 5.20 | — |
| 66 | 94.00 | — | 0.20 | — | — | 5.80 | — |
| 67 | 94.20 | Trace | — | — | — | 5.80 | — |
| 68 | 91.70 | Trace | 2.30 | — | 0.40 | 5.60 | — |
| 69 | 92.30 | Trace | 2.10 | — | 0.30 | 5.30 | — |
| 70 | 92.50 | Trace | 2.10 | — | 0.60 | 4.80 | — |
| 71 | 79.80 | 10.20 | 2.30 | — | — | 4.60 | 3.00 |
| 72 | 91.80 | 3.30 | — | — | — | 4.90 | — |

TABLE I (contd.)

Catalyst: $Co_2(CO)_8$+Ligand
Unsaturated Olefin Feed: Acrylonitrile

| Example | Ligand | $Co_2(CO)_8$/ Ligand | Solvent | Temp (°C) | Pres (kg/cm²) | Time (min) | Conv (%) |
|---|---|---|---|---|---|---|---|
| 73 | 4-PcNox | 1:1 | DMP+MeOH | 97.5 | 21 | 180 | 91.10 |
| 74 | 4-PcNox | 1:2 | DMP+MeOH | 97.5 | 21 | 180 | 99.50 |
| 75 | 4-PcNox | 1:2 | DMP+MeOH | 117.5 | 21 | 25 | 58.80 |
| 76 | 4-PcNox | 1:1 | DMP+MeOH | 117.5 | 21 | 120 | 38.44 |
| 77 | 4-PcNox | 1:8 | DMP+MeOH | 117.5 | 21 | 120 | 45.30 |
| 78 | 4-PcNox | 1:2 | DMP+MeOH | 117.5 | 21 | 150 | 63.30 |
| 79 | 4-PcNox | 1:4 | DMP+MeOH | 117.5 | 21 | 180 | 87.40 |
| 80 | 4-PcNox | 1:8 | DMP+MeOH | 117.5 | 21 | 180 | 48.20 |
| 81 | PyNox | 1:2 | MeOH | 95 | 56 | 60 | 100.00 |
| 82 | PyNox | 1:2 | MeOH | 97.5 | 56 | 180 | 100.00 |
| 83 | 2-PcNox | 1:2 | MeOH | 95 | 56 | 120 | 87.03 |
| 84 | 4-MPyNox | 1:2 | MeOH | 95 | 56 | 90 | 100.00 |
| 85 | 4-MPyNox | 1:2 | DMP+MeOH | 97.5 | 28 | 20 | 61.13 |
| 86 | 4-MPyNox | 1:2 | DMP+MeOH | 97.5 | 56 | 10 | 69.40 |
| 87 | 4-MPyNox | 1:2 | DMP+MeOH | 97.5 | 56 | 20 | 94.30 |
| 88 | 4-MPyNox | 1:4 | MeOH | 97.5 | 56 | 90 | 97.40 |
| 89 | 4-MPyNox | 1:16 | MeOH | 97.5 | 56 | 180 | 71.30 |
| 90 | Pc acid | 1:2 | MeOH | 97.5 | 56 | 180 | 42.30 |
| 91 | 3-Py Carb | 1:2 | DMP+MeOH | 97.5 | 56 | 15 | 57.70 |
| 92 | 3-Py Carb | 1:2 | DMP+MeOH | 97.5 | 56 | 30 | 95.20 |
| 93 | 3-Py Carb | 1:2 | MeOH | 97.5 | 56 | 180 | 100.00 |
| 94 | QuNox | 1:2 | MeOH | 97.5 | 56 | 180 | 100.00 |
| 95 | t-but PyNox | 1:2 | DMP+MeOH | 97.5 | 56 | 25 | 81.40 |
| 96 | t-but PyNox | 1:2 | DMP+MeOH | 97.5 | 56 | 60 | 100.00 |
| 97[1] | t-but PyNox | 1:2 | MeOH | 97.5 | 56 | 180 | 100.00 |

TABLE I (contd.)

| | Yields (%) | | | | | | |
|---|---|---|---|---|---|---|---|
| Example | 3-CE | 2-CE | PN | 3-CPA | 3-CPAA | 3-MPN | Other |
| 73 | 72.20 | 18.20 | 5.30 | — | — | 4.30 | — |
| 74 | 76.80 | 16.90 | 2.00 | — | 0.20 | 4.20 | — |
| 75 | 82.40 | — | 9.70 | — | — | 7.90 | — |
| 76 | 77.32 | — | 11.57 | — | — | 5.75 | 5.35 |
| 77 | 78.60 | — | 11.60 | — | — | 7.50 | 2.40 |
| 78 | 80.70 | — | 8.30 | — | 0.30 | 10.80 | — |
| 79 | 93.40 | 6.00 | — | — | — | 0.50 | — |
| 80 | 69.60 | — | 13.20 | — | — | 13.60 | 3.70 |
| 81 | 31.58 | 62.38 | 2.14 | — | 3.54 | 0.06 | 0.31 |
| 82 | 52.40 | 42.20 | 1.50 | — | 3.10 | — | — |
| 83 | 8.99 | 78.75 | 2.13 | 3.43 | 2.71 | 0.32 | 3.66 |
| 84 | 45.68 | 51.96 | 0.91 | — | 0.99 | 0.19 | 0.27 |
| 85 | 84.07 | 10.00 | 2.16 | — | — | — | 3.77 |
| 86 | 65.50 | 30.50 | — | — | 4.00 | — | — |
| 87 | 55.50 | 41.00 | 1.00 | — | 2.50 | — | — |
| 88 | 34.50 | 62.30 | 0.99 | — | 0.28 | 2.10 | 0.12 |
| 89 | 19.80 | 72.90 | 2.84 | — | — | 1.37 | 3.07 |
| 90 | 24.10 | — | — | — | 6.90 | 23.50 | 45.40 |
| 91 | 69.10 | 30.90 | — | — | — | — | — |
| 92 | 41.90 | 46.30 | 0.90 | 6.70 | 4.20 | — | — |
| 93 | 40.50 | 52.80 | 1.95 | — | 4.40 | 0.03 | 8.28 |
| 94 | 1.09 | 93.30 | — | 0.67 | 4.58 | 0.07 | 0.27 |
| 95 | 58.30 | 28.40 | — | — | — | — | 13.30 |
| 96 | 40.80 | 51.50 | 0.20 | — | — | — | 7.30 |
| 97[1] | 50.50 | 46.80 | 1.30 | — | 0.60 | 0.30 | 0.40 |

[1]2.2% $H_2$
[2]10% $H_2$
[3]2×Cat. Conc.
[4]0% $H_2$
[5]Preformed in 5% $H_2$; run on 0% $H_2$
[6]1% $H_2$
[7]1/10×Cat. Conc.
[8]1/2 Cat. Conc.

Compound name abbreviations

| Abbreviation | Compound name |
|---|---|
| ADN | adiponitrile |
| BiPy-Nox | bipyridyl-di-N-oxide |
| 2 CE | methyl-$\alpha$-cyanopropionate |
| 3 CE | methyl-$\beta$-cyanopropionate |
| 3 CPA | 3-cyano-propionaldehyde |
| 3 CPAA | 3-cyano-propionaldehyde dimethyl acetal |
| DIABLO | diazabicyclo (2.2.2) ocatane |
| DMP | dimethylphthalate |
| DMSO | dimethyl sulphoxide |
| 3-MPN | 3-methoxy propionitrile |
| 4-MPyNox | 4-methoxy-pyridine-N-oxide |
| 4-NPyNox | 4-nitro-pyridine-N-oxide |
| Pc acid | picolinic acid-N-oxide |
| 2-PcNox | 2-picoline-N-oxide |
| 4-PcNox | 4-picoline-N-oxide |
| PN | propionitrile |
| PVPYRL | polyvinylpyrrolidone |
| 3-Py Carb | 3-pyridylcarbinol-N-oxide |
| PyNox | pyridine-N-oxide |
| QuNox | quinoline-N-oxide, dihydrate |
| TAD | tetraazadecane |
| t-but PyNox | 4-t-butyl-pyridine-N-oxide |
| TEPO | triethyl phosphate oxide |
| TPPO | triphenylphosphine oxide |

Example 101

In each of the above examples, methanol was used as the compound containing a replaceable hydrogen atom. In the following examples, either t-butyl alcohol or n-amyl alcohol were used in place of methanol.

13.5 g of acrylonitrile, 0.88 g of 4-picoline-N-oxide and 100 ml of t-butyl alcohol were placed into a glasslined autoclave. Next, 1.37 g of $Co_2(CO)_8$ were added and the autoclave sealed.

The autoclave was charged with synthesis gas containing 5% $H_2$ to a pressure of 56 kg/cm$^2$ (800 psi). The temperature was set at 97.5°C and the reaction proceeded for 180 minutes. The glasslined autoclave was then brought to room temperature by cooling with cold water, depressurized and opened for product analysis. The product analysis is shown in Table II.

Examples 102 and 103

These examples follow the same procedure outlined in Example 101 except that the solvent, alcohol and reaction time were varied. These variables and the product analysis are shown in Table II.

Examples 104

In each of the above examples the unsaturated olefin feed was acrylonitrile. In the following two examples, other olefins were fed to the inventive reaction system.

5.26 g of propylene, 0.88 g of 4-picoline-N-oxide, 8.15 g of methanol and 100 ml of orthoxylene were placed into a glasslined autoclave. Next, 1.37 g of $Co_2(CO)_8$ were added and the autoclave sealed.

The autoclave was charged with synthesis gas containing 5% $H_2$ to a pressure of 56 kg/cm$^2$ (800 psi). The temperature was set at 97.5°C and the reaction proceeded for 180 minutes. The glasslined autoclave was then brought to room temperature by cooling with cold water, depressurized and opened for product analysis. An 80% conversion of propylene was obtained with a 56% yield of N-methylbutyrate and a 20% yield of methyl isobutyrate.

Example 105

14.8 g of allyl alcohol, 1.45 g of quinoline-N-oxide and 100 ml of t-butyl alcohol were placed into a glasslined autoclave. Next, 1.37 g of $Co_2(CO)_8$ were added and the autoclave sealed.

The autoclave was charged with synthesis gas containing 5% $H_2$ to a pressure of 56 kg/cm$^2$ (800 psi). The temperature was set at 97.5°C and the reaction proceeded for 180 minutes. The glasslined autoclave was then brought to room temperature by cooling with cold water, depressurized and opened for product analysis. A 100% conversion of allyl alcohol was obtained with a 50% yield of propionaldehyde and a 50% yield of butyrolactone.

Example 106

Each of the above examples used a heterocyclic nitrogen ligand. The following examples use phosphorus or sulphur oxide ligands.

13.5 g of acrylonitrile, 1.46 of triethylphosphateoxide and 100 ml of methanol were placed into a glasslined autoclave. Next, 1.37 g of $Co_2(CO)_8$ were added and the autoclave sealed.

The autoclave was charged with synthesis gas containing 5% $H_2$ to a pressure of 56 kg/cm$^2$ (800 psi). The temperature was set at 95°C and the reaction proceeded for 180 minutes. The glasslined autoclave was then brought to room temperature by cooling with cold water, depressurized and opened for product analysis. The product analysis is shown in Table III.

Examples 107—109

The procedure outlined in Example 106 was followed with the temperature, reaction time and ligand being varied. These variables and the product analysis are shown in Table III.

TABLE II
Carbonylation with heterocyclic nitrogen ligand

Catalyst: $Co_2(CO)_8$+ligand, unsaturated olefin feed: acrylonitrile

| Example | Ligand | $Co_2(CO)_8$/ Ligand | Solvent | Temp (°C) | Pres kg/cm$^2$ | Time (min) | Conv (%) |
|---|---|---|---|---|---|---|---|
| 101 | 4-PcNox | 1:2 | t-but alcohol | 97.5 | 56 | 180 | 100.00 |
| 102 | 4-PcNox | 1:2 | ADN+t-but alcohol | 97.5 | 56 | 150 | 43.72 |
| 103 | 4-PcNox | 1:2 | ADN+n-amyl alcohol | 97.5 | 56 | 180 | 55.62 |

TABLE II (contd.)

| | Yields (%) | | | | | | |
|---|---|---|---|---|---|---|---|
| Example | 3-CE | 2-CE | PN | 3-CPA | 3-CPAA | 3-MPN | Other |
| 101 | 1.80 | 94.90 | 1.50 | 0.75 | — | — | 1.20 |
| 102 | — | 87.39 | — | — | — | — | 12.61 |
| 103 | 39.70 | 0.61 | 1.99 | — | 0.68 | 10.19 | 46.83 |

TABLE III
Carbonylation with phosphorus or sulphur alkyl oxides

Catalyst: $Co_2(CO)_8$+ligand unsaturated olefin feed: acrylonitrile

| Example | Ligand | $Co_2(CO)_8$/ Ligand | Solvent | Temp (°C) | Pres kg/cm² | Time (min) | Conv (%) |
|---|---|---|---|---|---|---|---|
| 106 | TEPO | 1:2 | MeOH | 95 | 56 | 180 | 65.30 |
| 107 | DMSO | 1:2 | MeOH | 95 | 56 | 180 | 64.10 |
| 108 | TPPO | 1:2 | MeOH | 115 | 56 | 90 | 57.20 |
| 109 | TPPO | 1:2 | MeOH | 95 | 56 | 240 | 76.70 |

TABLE III (contd.)

| | Yields (%) | | | | | | |
|---|---|---|---|---|---|---|---|
| Example | 3-CE | 2-CE | PN | 3-CPA | 3-CPAA | 3-MPN | Other |
| 106 | 4.87 | 64.50 | 9.60 | — | 9.39 | — | 11.64 |
| 107 | 3.83 | 57.70 | 10.84 | — | 12.70 | 2.73 | 12.15 |
| 108 | 4.81 | 44.30 | 18.70 | — | 8.56 | 10.50 | 13.15 |
| 109 | 6.05 | 72.70 | 7.70 | — | 7.05 | 9.13 | 5.59 |

## Claims

1. A catalyst composition comprising a promoter ligand which is a heterocyclic nitrogen compound or a phosphorus or a sulphur oxide and a carbonyl of cobalt and/or ruthenium characterised in that

(1) the heterocyclic nitrogen promoter ligand has the following structure:

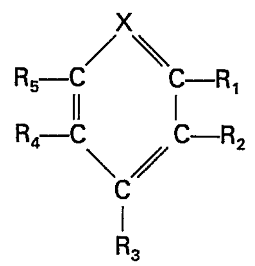

wherein X is NO; and wherein $R_1$, $R_2$, $R_3$, $R_4$ and $R_5$ are each independently selected from:
(1) H;
(2) $C_{1-10}$ alkyls;
(3) $(CH_2)_q$ OH wherein q is 0—10;

(4) 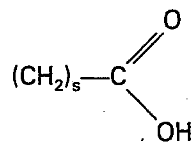

wherein s is 0—10; and

(5) $O(CH_2)_tCH_3$ wherein t is 0—10; and wherein $R_1$ and $R_2$ may comprise a five to eight membered carboxyclic fused ring optionally substituted with $C_{1-10}$ alkyls; or

(2) the phosphorus or sulphur oxide promoter ligand has one of the following structures:

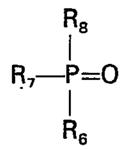

and

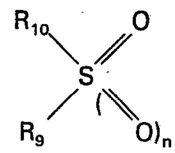

wherein $R_6$, $R_7$, $R_8$, $R_9$ and $R_{10}$ are each independent selected from:

(1) $C_1$10 alkyls;
(2) polynuclear aryls containing up to 12 carbon atoms, optionally substituted with $C_{1-10}$ alkyls; and
(3) $O(CH_2)_tCH_3$, wherein t is 0—10; and wherein n is 0 or 1.

2. A catalyst composition as claimed in claim 1 characterised in that $R_1$, $R_2$, $R_3$, $R_4$ and $R_5$ in the heterocyclic nitrogen promoter ligand are each independently selected from:

(1) H;
(2) $C_{1-4}$ alkyls;
(3) $(CH_2)_qOH$ wherein q is 0 or 1;

(4) 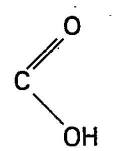 ; and

(5) $OCH_3$.

3. A catalyst composition as claimed in claim 1 characterised in that $R_6$, $R_7$, $R_8$, $R_9$ and $R_{10}$ in the phosphorus or sulphur oxide promoter ligand are each independently selected from:

(1) $C_{1-4}$ alkyls;
(2) aryls, optionally substituted with $C_{1-4}$ alkyls; and
(3) $OCH_3$.

4. A catalyst composition as claimed in any of claims 1 to 3 characterised in that the ligand and the cobalt or ruthenium carbonyl are mixed with an inert organic solvent.

5. A process for the production of catalysts as claimed in any of claims 1 to 4 in which the cobalt and/or ruthenium carbonyl is mixed with at least one promoter ligand in a solvent.

6. A process for the production of an oxygenated organic compound comprising contacting an olefinically unsaturated compound with carbon monoxide and a compound containing a replaceable hydrogen atom in the presence of a catalyst as claimed in any of claims 1 to 4 or prepared according to claim 5.

7. A process as claimed in claim 6 characterised in that the olefinically unsaturated compound has the following structure:

$$R_{11}CH{=}CHR_{12}$$

wherein $R_{11}$ and $R_{12}$ are each independently selected from:

(1) hydrogen (either $R_{11}$ or $R_{12}$ but not both);
(2) $C_{1-30}$ alkyl;
(3) $-(CH_2)_p-CN$, wherein p is 0—3; and
(4) $-(CH_2)_q-OR_{13}$, wherein q is 1—30 and $R_{13}$ is hydrogen or methyl.

8. A process as claimed in claim 7 characterised in that the olefinically unsaturated compound is acrylonitrile, propylene, methacrylate, allyl alcohol and/or pentenenitrile.

9. A process as claimed in any of claims 6 to 8 characterised in that the compound containing a replaceable hydrogen atom is represented by the following formula:

$$H—Y$$

wherein Y is selected from:

(1) $OR_{14}$ wherein $R_{14}$ is a $C_{1-30}$ alkyl;

(2) 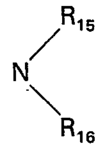

wherein $R_{15}$ and $R_{16}$ are each independently selected from $C_{1-10}$ alkyls; and

(3) H.

10. A process as claimed in claim 9 characterised in that the compound containing a replaceable hydrogen atom is methanol, ethanol, propanol and/or butanol.

11. A process as claimed in claim 9 characterised in that the compound containing a replaceable hydrogen atom is $H_2$.

12. A process for the production of an oxygenated organic compound comprising contacting an olefinically unsaturated compound containing an alcohol moiety with carbon monoxide in the presence of a catalyst as claimed in any of claims 1 to 4 or prepared according to claim 5.

13. A process as claimed in claim 12 characterised in that the olefinically unsaturated compound has the following structure:

$$R_{11}CH{=}CHR_{12}$$

wherein $R_{11}$ and $R_{12}$ are each independently selected from:

(1) hydrogen;
(2) $C_{1-30}$ alkyl;
(3) $—(CH_2)_p—CN$, wherein p is 0—3; and
(4) $—(CH_2)_q—OH$, wherein q is 1—30; with the proviso that at least one of $R_{11}$ and $R_{12}$ is an alcohol moiety.

14. A process as claimed in claim 13 characterised in that $H_2$ is present in the reaction system.

15. A process as claimed in any of claims 6 to 14 characterised in that it is carried out in the presence of an inert organic solvent.

16. A process for the separation of a substantially active catalyst, which catalyst has the composition defined in claim 1 from an oxygenated cyano compound product contained in a reaction mixture having a solvent selected from the group of aromatics, substituted aromatics, esters, nitriles, and mixtures thereof comprising the steps of:

contacting the reaction mixture with a hydrocarbon having from 5 to 8 carbon atoms to form a hydrocarbon/solvent conjugate solution, wherein the hydrocarbon is at least partially miscible with the solvent; and

recovering the solvent phase from the hydrocarbon phase of the conjugate solution, whereby said active catalyst remains substantially in the solvent phase.

17. A process as claimed in claim 16 characterised in that the volumetric ratio of hydrocarbon to reaction mixture is 1:1 to 4:1.

**Revendications**

1. Une composition catalytique comprenant un ligand promoteur qui est un composé azoté hétérocyclique ou un oxyde de phosphore ou du soufre et un dérivé carbonylé de cobalt et/ou de ruthénium, caractérisée en ce que

(1) le ligand promoteur azoté hétérocyclique a la structure suivante:

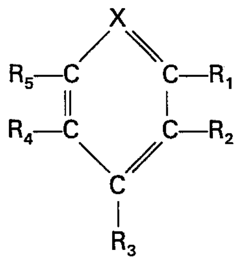

où X est NO; et
où $R_1$, $R_2$, $R_3$, $R_4$, et $R_5$ sont choisis chacun indépendamment parmi:

(1) H;
(2) des groupes alcoyle en $C_{1-10}$;
(3) $(CH_2)_qOH$ où q est 0—10

(4) 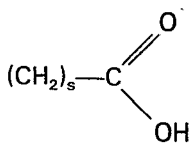 où s est 0—10; et

(5) $O(CH_2)_tCH_3$ où t est 0—10; et où $R_1$ est $R_2$ peuvent comprendre un noyau carbocyclique condensé de cinq à huit chaînons éventuellement substitué par des groupes alcoyle en $C_{1-10}$; ou

(2) le ligand promoteur oxyde du phosphore ou du soufre a l'une des structures suivantes:

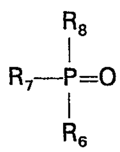 et 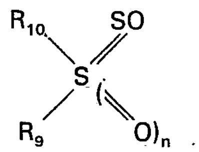 .

où $R_6$, $R_7$, $R_8$, $R_9$ et $R_{10}$ sont choisis chacun indépendamment parmi:

(1) des groupes alcoyle en $C_{1-10}$;
(2) des groupes aryle polycycliques contenant jusqu'à 12 atomes de carbone, éventuellement substitués par des groupes alcoyle en $C_{1-10}$; et
(3) $O(CH_2)_tCH_3$, où t est 0—10; et où n est 0 ou 1.

2. Une composition catalytique selon la revendication 1, caractérisé en ce que $R_1$, $R_2$, $R_3$, $R_4$ et $R_5$ dans le ligand promoteur azoté hétérocyclique sont choisis chacun indépendamment parmi:

(1) H;
(2) des groupes alcoyle en $C_{1-4}$;
(3) $(CH_2)_qOH$ où q est 0 et 1;

(4) 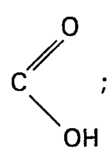 ; et

(5) $OCH_3$.

3. Une composition catalytique selon la revendication 1, caractérisée en ce que $R_6$, $R_7$, $R_8$, $R_9$ et $R_{10)}$ dans le ligand promoteur oxyde du phosphore ou du soufre sont choisis chacun indépendamment parmi:

(1) des groupes alcoyle en $C_{1-4}$;
(2) des groupes aryle, éventuellement substitués par des groupes alcoyle en $C_{1-4}$; et
(3) $OCH_3$.

4. Une composition catalytique selon l'une quelconque des revendications 1 à 3, caractérisée en ce que le ligand et le cobalt ou ruthénium carbonyle sont mélangés avec un solvant organique inerte.

5. Un procédé pour la production de catalyseurs tels que définis dans l'une quelconque des revendications 1 à 4, dans lequel le cobalt et/ou le ruthénium carbonyle sont mélangés avec au moins un ligand promoteur dans un solvant.

6. Un procédé pour la production d'un composé organique oxygéné comprenant la mise en contact d'un composé oléfiniquement insaturé avec l'oxyde de carbone et un composé contenant un atome d'hydrogène remplaçable en présence d'un catalyseur tel que défini dans l'une quelconque des revendications 1 à 4 ou préparé selon la revendication 5.

7. Un procédé selon la revendication 6, caractérisé en ce que le composé oléfiniquement insaturé a la structure suivante:

$$R_{11}CH{=}CHR_{12}$$

où $R_{11}$ et $R_{12}$ sont choisis chacun indépendamment parmi:

(1) l'hydrogène ($R_{11}$ ou $R_{12}$, mais pas les deux);
(2) un groupe alcoyle en $C_{1-30}$;
(3) $—(CH_2)_p—CN$, où p est 0—3; et
(4) $—(CH_2)_q—OR_{13}$, où q est 1—30 et $R_{13}$ est de l'hydrogène ou un groupe méthyle.

8. Un procédé selon la revendication 7, caractérisé en ce que le composé oléfiniquement insaturé est de l'acrylonitrile, du propylène, un méthacrylate, de l'alcool allylique et/ou du pentènenitrile.

9. Un procédé selon l'une quelconque des revendications 6 à 8, caractérisé en ce que le composé contenant un atome d'hydrogène remplaçable est représenté par la formule suivante:

$$H—Y$$

où Y est choisi parmi:

(1) $OR_{14}$ où $R_{14}$ est un groupe alcoyle en $C_{1-30}$;

(2) 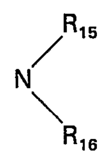

où $R_{15}$ et $R_{16}$ sont choisis chacun indépendamment parmi des groupes alcoyle en $C_{1-10}$; et
(3) H.

10. Un procédé selon la revendication 9, caractérisé en ce que le composé contenant un atome d'hydrogène remplaçable est du méthanol, de l'éthanol, du propanol et/ou du butanol.

11. Un procédé selon la revendication 9, caractérisé en ce que le composé contenant un atome d'hydrogène remplaçable est $H_2$.

12. Un procédé pour la production d'un composé organique oxygéné, comprenant la mise en contact d'un composé oléfiniquement insaturé contenant une portion alcool avec l'oxyde de carbone en présence d'un catalyseur tel que défini dans l'une quelconque des revendications 1 à 4 ou préparé selon la revendication 5.

13. Un procédé selon la revendication 12, caractérisé en ce que le composé oléfiniquement insaturé a la structure suivante:

$$C_{11}CH{=}CHR_{12}$$

où $R_{11}$ et $R_{12}$ sont choisis chacun indépendamment parmi:

(1) l'hydrogène;
(2) un groupe alcoyle en $C_{1-30}$;
(3) $—(CH_2)_p—CN$, où p est 0—3; et
(4) $—(CH_2)_q—OH$, où q est 1—30;

avec la condition qu'au moins une des portions $R_{11}$ et $R_{12}$ est une portion alcool.

14. Un procédé selon la revendication 13, caractérisé en ce que $H_2$ est présent dans le système de réaction.

15. Un procédé selon l'une quelconque des revendications 6 à 14, caractérisé en ce qu'il est mis en oeuvre en présence d'un solvant organique inerte.

16. Un procédé pour séparer un catalyseur nettement actif, ce catalyseur ayant la composition

définie dans la revendication 1, d'un produit cyané oxygéné contenu dans un mélange de réaction comportant un solvant choisi parmi des composés aromatiques, des composés aromatiques substitués, des esters, des nitriles et leurs mélanges, comprenant les étapes selon lesquelles:

on met en contact le mélange de réaction avec un hydrocarbure ayant de 5 à 8 atomes de carbone pour former une solution conjuguée hydrocarbure/solvant, l'hydrocarbure étant au moins partiellement miscible avec le solvant: et

on recueille la phase de solvant à partir de la phase hydrocarbonée de la solution conjuguée, de façon que le catalyseur actif reste essentiellement dans la phase de solvant.

17. Un procédé selon la revendication 16, caractérisé en ce que le rapport volumétrique de l'hydrocarbure au mélange de réaction est compris entre 1:1 et 4:1.

**Patentansprüche**

1. Katalysatorzusammensetzung, die umfaßt einen Promotor-Liganden, bei dem es sich um eine heterocyclische Stickstoffverbindung oder ein Phosphor- oder Schwefeloxid handelt, und ein Carbonyl von Kobalt und/oder Ruthenium, dadurch gekennzeichnet, daß

(1) der heterocyclische Stickstoff-Promotor-Ligand die folgende Struktur hat:

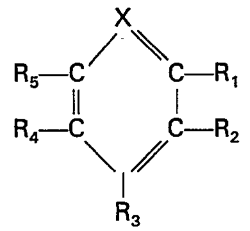

worin X NO bedeutet und
worin $R_1$, $R_2$, $R_3$, $R_4$ und $R_5$ jeweils unabhängig voneinander ausgewählt werden aus:

(1) H
(2) $C_{1-10}$-Alkylen;
(3) $(CH_2)_q$ OH, worin q 0 bis 10 darstellt;

(4) 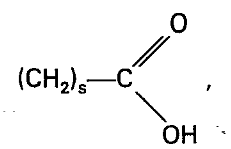 ,

worin s 0 bis 10 darstellt; und
(5) $O(CH_2)_tCH_3$, worin t 0 bis 10 darstellt; und

worin $R_1$ und $R_2$ einen 5- bis 8-gliedrigen carbocyclischen kondensierten Ring bilden können, der gegebenenfalls durch $C_{1-10}$-Alkyle substituiert ist; oder
(2) der Phosphor- oder Schwefeloxid-Promotor-Ligand eine der folgenden Strukturen hat:

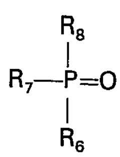

und

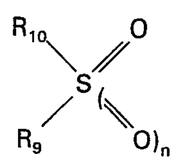

worin $R_6$, $R_6$, $R_7$, $R_8$, $R_9$ und $R_{10}$ jeweils unabhängig voneinander ausgewählt werden aus:
(1) $C_{1-10}$-Alkylen;
(2) polynuklearen Arylen, die bis zu 12 Kohlenstoffatome enthalten und gegebenenfalls substituiert sind durch $C_{1-10}$-Alkyle; und
(3) $O(CH_2)_tCH_3$, worin t 0 bis 10 darstellt; und
worin n die Zahl 0 oder 1 bedeutet.

2. Katalysatorzusammensetzung nach Anspruch 1, dadurch gekennzeichnet, daß $R_1$, $R_2$, $R_3$, $R_4$ und $R_5$ in dem heterocyclischen Stickstoff-Promotor-Liganden jeweils unabhängig voneinander ausgewählt werden aus:

(1) H;
(2) $C_{1-4}$-Alkylen;
(3) $(CH_2)_qOH$, worin q 0 oder 1 darstellt;

(4) 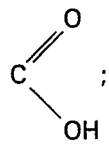 ; und

(5) $OCH_3$.

3. Katalysatorzusammensetzung nach Anspruch 1, dadurch gekennzeichnet, daß $R_6$, $R_7$, $R_8$, $R_9$ und $R_{10}$ in dem Phosphor- oder Schwefeloxid-Promotor-Ligand jeweils unabhängig voneinander ausgewählt werden aus:

(1) $C_{1-4}$-Alkylen;
(2) Arylen, die gegebenenfalls substituiert sind durch $C_{1-4}$-Alkyle; und
(3) $OCH_3$.

4. Katalysatorzusammensetzung nach einem der Ansprüche 1 bis 3, dadurch gekennzeichnet, daß der Ligand und das Kobalt- oder Rutheniumcarbonyl mit einem inerten organischen Lösungsmittel gemischt sind.

5. Verfahren zur Herstellung der Katalysatoren nach einem der Ansprüche 1 bis 4, bei dem das Kobalt- und/oder Rutheniumcarbonyl mit mindestens einem Promotor-Liganden in einem Lösungsmittel gemischt wird.

6. Verfahren zur Herstellung einer sauerstoffhaltigen organischen Verbindung, das umfaßt das Inkontaktbringen einer olefinisch ungesättigten Verbindung mit Kohlenmonoxid und einer Verbindung, die ein ersetzbares Wasserstoffatom enthält, in Gegenwart eines Katalysators nach einem der Ansprüche 1 bis 4 oder hergestellt nach Anspruch 5.

7. Verfahren nach Anspruch 6, dadurch gekennzeichnet, daß die olefinisch ungesättigte Verbindung die folgende Struktur hat:

$$R_{11}CH{=}CHR_{12}$$

worin $R_{11}$ und $R_{12}$ jeweils unabhängig voneinander ausgewählt werden aus:

(1) Wasserstoff (entwerder $R_{11}$ oder $R_{12}$, jedoch nicht beide);
(2) $C_{1-30}$-Alkyl;
(3) $-(CH_2)_p-CN$, worin p 0 bis 3 darstellt; und
(4) $-(CH_2)_q-OR_{13}$, worin q 1 bis 30 und $R_{13}$ Wasserstoff oder Methyl darstellen.

8. Verfahren nach Anspruch 7, dadurch gekennzeichnet, daß es sich bei der olefinisch ungesättigten Verbindung um Acrylnitril, Propylen, Methacrylat, Allylalkohol und/oder Pentennitril handelt.

9. Verfahren nach einem der Ansprüche 6 bis 8, dadurch gekennzeichnet, daß die ein ersetzbares Wasserstoffatom enthaltende Verbindung dargestellt wird durch die folgende Formel:

$$H{-}Y$$

worin Y ausgewählt wird aus:

(1) $OR_{14}$, worin $R_{14}$ ein $C_{1-30}$-Alkyl darstellt;

(2) 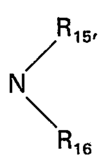

worin $R_{15}$ und $R_{16}$ jeweils unabhängig voneinander ausgewählt werden aus $C_{1-10}$-Alkylen; und
(3) H.

10. Verfahren nach Anspruch 9, dadurch gekennzeichnet, daß es sich bei der ein ersetzbares Wasserstoffatom enthaltenden Verbindung um Methanol, Ethanol, Propanol und/oder Butanol handelt.

11. Verfahren nach Anspruch 9, dadurch gekennzeichnet, daß es sich bei der ein ersetzbares Wasserstoffatom enthaltenden Verbindung um $H_2$ handelt.

12. Verfahren zur Herstellung einer sauerstoffhaltigen organischen Verbindung, das umfaßt das Inkontaktbringen einer einen Alkoholrest enthaltenden olefinisch ungesättigten Verbindung mit Kohlenmonoxid in Gegenwart eines Katalysators nach einem der Ansprüche 1 bis 4 oder hergestellt nach Anspruch 5.

13. Verfahren nach Anspruch 12, dadurch gekennzeichnet, daß die olefinisch ungesättigte Verbindung die folgende Struktur hat:

$$R_{11}CH{=}CHR_{12}$$

worin $R_{11}$ und $R_{12}$ jeweils unabhängig voneinander ausgewählt werden aus:

(1) Wasserstoff;
(2) $C_{1-30}$-Alkyl;
(3) $-(CH_2)_p-CN$, worin p 0 bis 3 darstellt; und
(4) $-(CH_2)_q-OH$, worin q 1 bis 30 darstellt;

mit der Maßgabe, daß mindestens einer der Reste $R_{11}$ und $R_{12}$ ein Alkoholrest ist.

14. Verfahren nach Anspruch 13, dadurch gekennzeichnet, daß $H_2$ in dem Reaktionssystem vorhanden ist.

15. Verfahren nach einem der Ansprüche 6 bis 14, dadurch gekennzeichnet, daß es in Gegenwart eines inerten organischen Lossungsmittels durchgeführt wird.

16. Verfahren zur Abtrennung eines im wesentlichen aktiven Katalysators, der die in Anspruch 1 angegebene Zusammensetzung hat, von einem sauerstoffhaltigen Cyanoverbindungsprodukt, das in einer Reaktionsmischung mit einem Lösungsmittel, ausgewählt aus der Gruppe der Aromaten, der substituierten Aromaten, der Ester, Nitrile und Mischungen davon, enthalten ist, das die folgenden Stufen umfaßt:

Inkontaktbringen der Reaktionsmischung mit einem Kohlenwasserstoff mit 5 bis 8 Kohlenstoffatomen unter Bildung einer Kohlenwasserstoff/Lösungsmittel-Konjugatlösung, worin der Kohlenwasserstoff mindestens teilweise mit dem Lösungsmittel mischbar ist; und

Abtrennen der Lösungsmittelphase von der Kohlenwasserstoffphase der Konjugatlösung, so daß der aktive Katalysator im wesentlichen in der Lösungsmittelphase verbleibt.

17. Verfahren nach Anspruch 16, dadurch gekennzeichnet, daß das volumetrische Verhältnis von Kohlenwasserstoff zur Reaktionsmischung 1:1 bis 4:1 beträgt.